# EUROPÄISCHE PATENTANMELDUNG

(11) **EP 3 557 270 A1**
(43) Veröffentlichungstag der Anmeldung: **23.10.2019**
(21) Anmeldenummer: 18167986.1
(22) Anmeldetag: 18.04.2018
(51) Int. Cl.: G01R 33/00, G12B 17/02, G01R 33/28, A61B 5/055

(54) **MAGNETRESONANZSCHUTZVORRICHTUNG MIT EINEM SCHIRMGEHÄUSE SOWIE EIN MAGNETRESONANZSYSTEM MIT EINER MAGNETRESONANZSCHUTZVORRICHTUNG**

(71) Anmelder: Siemens Healthcare GmbH, 91052 Erlangen (DE)
(72) Erfinder: Schneider, Rainer, 91054 Erlangen (DE)

(57) **Zusammenfassung**

Die Erfindung geht aus von einer Magnetresonanzschutzvorrichtung mit einem Schirmgehäuse und einen von dem Schirmgehäuse umschlossenen Bereich, der zu einer Aufnahme eines mobilen Endgeräts ausgelegt ist, wobei das Schirmgehäuse für elektromagnetischer Hochfrequenzstrahlung mit einer Frequenz von kleiner als 1,5 GHz undurchlässig und/oder nichttransparent ausgebildet ist.

## Beschreibung

Die vorliegende Erfindung betrifft eine Magnetresonanzschutzvorrichtung mit einem Schirmgehäuse, das zu einer Aufnahme eines mobilen Endgeräts ausgelegt ist, und einen von dem Schirmgehäuse umschlossenen Bereich. Des Weiteren geht die Erfindung aus von einem Magnetresonanzsystem mit einer Magnetresonanzvorrichtung und einer Magnetresonanzschutzvorrichtung.

Für Magnetresonanzuntersuchungen kann ein medizinisches Bedienpersonal meist an einem Display am Scanner einer Magnetresonanzvorrichtung Einstellungen für die Magnetresonanzuntersuchung eingeben. Zudem kann das medizinische Bedienpersonal Bilddaten an einem Monitor und/oder einem Display einer Benutzerschnittstelle einsehen. Aufgrund der unterschiedlichen Displays und Monitore muss sich das medizinische Bedienpersonal an die jeweiligen Einstellungen anpassen.

Auch dem Patienten kann während der Magnetresonanzuntersuchung ein Display für eine Mitteilung von Anweisungen und/oder zur Unterhaltung zur Verfügung stehen. Auch hier muss sich der Patienten an den Einstellungen des vorhandenen Displays anpassen.

Derartige Verfahren und Vorrichtungen sind beispielsweise aus DE 10 2015 201 521 A1 und DE 10 2015 211 331 A1 bekannt.

Der vorliegenden Erfindung liegt insbesondere die Aufgabe zugrunde, eine einfache Integration eines mobilen Endgeräts eines Benutzers in ein Magnetresonanzsystem zu ermöglichen. Die Aufgabe wird durch die Merkmale der unabhängigen Ansprüche gelöst. Vorteilhafte Ausgestaltungen sind in den Unteransprüchen beschrieben.

Die Erfindung geht aus von einer Magnetresonanzschutzvorrichtung mit einem Schirmgehäuse und einem von dem Schirmgehäuse umschlossenen Bereich, der zu einer Aufnahme eines mobilen Endgeräts ausgelegt ist. Dabei kann es vorgesehen sein, dass das Schirmgehäuse für elektromagnetische Hochfrequenzstrahlung mit einer Frequenz von kleiner als 1,5 GHz undurchlässig und/oder nichttransparent ausgebildet ist.

Die Magnetresonanzschutzvorrichtung ist bevorzugt dazu ausgebildet, ein mobiles Endgerät, beispielsweise ein mobiles Endgerät eines Patienten und/oder eines medizinischen Bedienpersonals, bezüglich einer elektromagnetischen Hochfrequenzstrahlung von der Magnetresonanzvorrichtung zu abzuschirmen. Insbesondere ist hierbei die Magnetresonanzschutzvorrichtung dazu ausgebildet, ein mobiles Endgerät für eine Strahlung in einen magnetresonanzsensitiven Frequenzbereich mit einer Frequenz von kleiner als 1,5 GHz von der Magnetresonanzvorrichtung abzuschirmen. Hierzu weist die Magnetresonanzschutzvorrichtung das Schirmgehäuse auf, das den vom dem Schirmgehäuse umschlossenen Bereich abschirmt. Das Schirmgehäuse, insbesondere der von dem Schirmgehäuse umschlossene Bereich, ist zu einer Aufnahme eines mobilen Endgeräts ausgelegt und/oder vorgesehen. Vorzugsweise umfasst das Schirmgehäuse alle Eigenschaften zu einer Abschirmung eines mobilen Endgeräts von der Magnetresonanzvorrichtung bezüglich einer elektromagnetischen Hochfrequenzstrahlung, insbesondere in einen magnetresonanzsensitiven Frequenzbereich. Die Magnetresonanzschutzvorrichtung, insbesondere das Schirmgehäuse, bildet somit eine Schutzhülle und/oder Abschirmhülle um das mobile Endgerät. Zudem gewährt und/oder ermöglicht die Magnetresonanzschutzvorrichtung, insbesondere das Schirmgehäuse, eine mobile Verwendung des mobilen Endgeräts in einer Umgebung der Magnetresonanzvorrichtung.

Das Schirmgehäuse ist bevorzugt für eine elektromagnetische Hochfrequenzstrahlung mit einer Frequenz, die im Arbeitsbereich der Magnetresonanzvorrichtung angeordnet ist, undurchlässig und/oder intransparent ausgebildet. Das Schirmgehäuse ist bevorzugt für eine elektromagnetische Hochfrequenzstrahlung mit einer Frequenz von kleiner als 1,5 GHz undurchlässig und/oder intransparent ausgebildet. Besonders vorteilhaft ist das Schirmgehäuse für eine elektromagnetische Hochfrequenzstrahlung mit einer Frequenz von kleiner als 1,4 GHz undurchlässig und/oder intransparent ausgebildet. Besonders vorteilhaft ist das Schirmgehäuse für eine elektromagnetische Hochfrequenzstrahlung mit einer Frequenz von kleiner als 1,3 GHz undurchlässig und/oder intransparent ausgebildet. Besonders vorteilhaft ist das Schirmgehäuse für eine elektromagnetische Hochfrequenzstrahlung mit einer Frequenz von kleiner als 1,2 GHz undurchlässig und/oder intransparent ausgebildet. Besonders vorteilhaft ist das Schirmgehäuse für eine elektromagnetische Hochfrequenzstrahlung mit einer Frequenz von kleiner als 1,1 GHz undurchlässig und/oder intransparent ausgebildet. Besonders vorteilhaft ist das Schirmgehäuse für eine elektromagnetische Hochfrequenzstrahlung mit einer Frequenz von kleiner als 1,0 GHz undurchlässig und/oder intransparent ausgebildet.

Das mobile Endgerät kann dabei ein mobiles Endgerät eines medizinischen Bedienpersonals oder auch ein mobiles Endgerät eines Patienten umfassen. Das mobile Endgerät kann dabei eine Tablet-PC oder ein Smartphone usw. umfassen.

Durch die Erfindung kann vorteilhaft der von dem Schirmgehäuse umschlossene Bereich und damit auch ein mobiles Endgerät, das innerhalb des von dem Schirmgehäuse umschlossenen Bereichs angeordnet ist, für magnetresonanzsensitive Frequenzen abgeschirmt werden. Insbesondere blockiert und/oder verhindert das Schirmgehäuse eine Transmission von magnetresonanzsensitiven Frequenzen durch das Schirmgehäuse. Magnetresonanzsensitive Frequenzen sind dabei im Bereich von kleiner als 1,5 GHz, wie insbesondere kleiner als 1,3 GHz und bevorzugt kleiner als 1,1 GHz angeordnet.

Des Weiteren kann eine einfache Integration eines mobilen Endgeräts eines Benutzers in ein Magnetresonanzsystem ermöglicht werden. Insbesondere kann beispielsweise während einer Magnetresonanzuntersuchung der Patient sein mobiles Endgerät für eine Patientenunterhaltung und/oder zu einer Übermittlung von Anweisungen während der Magnetresonanzuntersuchung mit in den Untersuchungsraum nehmen. Dies kann auch zur Beruhigung des Patienten während der Magnetresonanzuntersuchung beitragen, da dem Patienten seine gewohnte Darstellung eines Unterhaltungsmediums zur Verfügung steht.

Zudem kann auch ein medizinisches Bedienpersonal sein individualisiertes mobiles Endgerät zur Kontrolle der Magnetresonanzuntersuchung mit in den Untersuchungsraum nehmen. Dies erleichtert beispielsweise einen Zugriff auf bereits voreingestellte und/oder vorinstallierte Einstellungen für einzelne Untersuchungsschritte. Hierdurch kann auch ein Untersuchungsablauf beschleunigt werden, da ein Zugriff auf personalisierte Einstellungen erleichtert werden kann.

Vorzugsweise umhüllt das Schirmgehäuse den umschlossenen Bereich zur Aufnahme des mobilen Endgeräts komplett und/oder vollständig, so dass eine vollständige Abschirmung zwischen den umschlossenen Bereich und der Magnetresonanzvorrichtung gewährleistet werden kann.

In einer vorteilhaften Weiterbildung der Erfindung kann es vorgesehen sein, dass das Schirmgehäuse einen optisch transparenten Bereich und einen optisch nichttransparenten Bereich umfasst. In diesem Zusammenhang soll unter einem optisch transparenten Bereich insbesondere ein Bereich verstanden werden, der für elektromagnetische Strahlung, die für das menschliches Auge im sichtbaren Bereich ist, transparent und/oder durchlässig ausgebildet ist. Vorzugsweise ist dieser Bereich für elektromagnetische Strahlung mit einer Frequenz, deren Wert zwischen 350 THz und 800 THz liegt, transparent und/oder durchlässig ausgebildet. Insbesondere ist dieser Bereich für sichtbares Licht durchlässig und/oder transparent ausgebildet. Zudem kann dieser optisch transparente Bereich auch für Infrarot-Strahlung durchlässig und/oder transparent ausgebildet sein, wie beispielsweise in einem Frequenzbereich für elektromagnetische Strahlung mit einer Frequenz, deren Wert zwischen 300 GHz und 400 THz liegt, transparent und/oder durchlässig ausgebildet sein. Vorzugsweise ist der optisch transparente Bereich zusätzlich für eine elektromagnetische Hochfrequenzstrahlung mit einer Frequenz von kleiner als 1,5 GHz undurchlässig und/oder intransparent ausgebildet.

Unter einem optisch nichttransparenten Bereich soll zudem ein Bereich verstanden werden, der für elektromagnetische Strahlung, die für das menschliches Auge im sichtbaren Bereich ist, nichttransparent und/oder undurchlässig ausgebildet ist. Vorzugsweise ist dieser Bereich für elektromagnetische Strahlung mit einer Frequenz, deren Wert zwischen 350 THz und 800 THz liegt, nichttransparent und/oder undurchlässig ausgebildet. Insbesondere ist dieser Bereich für sichtbares Licht undurchlässig und/oder nichttransparent ausgebildet.

Diese Ausgestaltung der Erfindung ermöglicht eine vorteilhafte Sichtbarkeit eines Displays und/oder eines Touch-Displays und/oder Monitors eines innerhalb des von dem Schirmgehäuse umschlossenen Bereichs befindlichen mobilen Endgeräts. Hierdurch kann neben einem vorteilhaften Schutz durch das Schirmgehäuse auch eine Funktionalität des mobilen Endgeräts für den Benutzer aufrechterhalten werden. Besonders vorteilhaft ist hierbei der optisch transparente Bereich zu einer Abschirmung einer Bildschirmoberfläche und/oder eines Displays und/oder eines Touch-Displays und/oder eines Monitors des mobilen Endgeräts ausgelegt.

In einer vorteilhaften Weiterbildung der Erfindung kann es vorgesehen sein, dass der optisch transparente Bereich eine optisch transparente Folie umfasst. Vorzugsweise ist die optisch transparente Folie für eine elektromagnetische Hochfrequenzstrahlung mit einer Frequenz von kleiner als 1,5 GHz undurchlässig und/oder intransparent ausgebildet. Mittels der Folie kann eine besonders kostengünstige Magnetresonanzschutzvorrichtung mit einem optisch transparenten Bereich bereitgestellt werden.

Alternativ oder zusätzlich kann der optisch transparente Bereich auch eine metallische Gitterstruktur umfassen. Die Metallische Gitterstruktur umfasst bevorzugt eine metallische Netzstruktur und/oder ein metallisches Netzgewebe. Die metallische Gitterstruktur kann dabei derart ausgebildet sein, dass die metallische Gitterstruktur für elektromagnetische Strahlung mit einer Frequenz, deren Wert zwischen 350 THz und 800 THz liegt, transparent und/oder durchlässig ist. Zudem kann die metallische Gitterstruktur dabei derart ausgebildet sein, dass die metallische Gitterstruktur für elektromagnetische Strahlung mit einer Frequenz von kleiner als 1,5 GHz undurchlässig und/oder intransparent ist. Hierdurch kann neben einem vorteilhaften Schutz durch das Schirmgehäuse auch eine Funktionalität des mobilen Endgeräts für einen Benutzer aufrechterhalten werden.

In einer vorteilhaften Weiterbildung der Erfindung kann es vorgesehen sein, dass das gesamte Schirmgehäuse eine metallische Gitterstruktur und/oder eine metallische Netzstruktur und/oder ein metallisches Netzgewebe umfasst. Hierdurch kann ein besonders kompaktes Schirmgehäuse bereitgestellt werden.

In einer vorteilhaften Weiterbildung der Erfindung kann es vorgesehen sein, dass der optisch transparente Bereich für eine Kommunikationsstrahlung mit einer Frequenz von größer als 1,8 GHz transparent ausgebildet ist. Vorzugsweise ist der optisch transparente Bereich für eine Kommunikationsstrahlung mit einer Frequenz von größer als 1,9 GHz transparent ausgebildet. Vorzugsweise ist der optisch transparente Bereich für eine Kommunikationsstrahlung mit einer Frequenz von größer als 2,0 GHz transparent ausgebildet. Vorzugsweise ist der optisch transparente Bereich für eine Kommunikationsstrahlung mit einer Frequenz von größer als 2,1 GHz transparent ausgebildet.

Unter eine Kommunikationsstrahlung mit einer Frequenz von größer als 1,8 GHz soll beispielsweise eine Bluetooth-Strahlung im Bereich von 2,4 GHz und/oder eine Wifi-Strahlung im Bereich von 2,4 GHz oder 5 GHz usw. verstanden werden. Die Kommunikationsstrahlung umfasst dabei eine elektromagnetische Strahlung, die zu einer Kommunikation und/oder einen Datenaustausch zwischen dem innerhalb des von dem Schirmgehäuse umschlossenen Bereichs angeordneten mobilen Endgeräts und der Magnetresonanzvorrichtung und/oder weiteren Einheiten vorgesehen ist. Dies ermöglicht einen vorteilhaften Datenaustausch mit beispielweise der Magnetresonanzvorrichtung und/oder einer Steuerungseinheit der Magnetresonanzvorrichtung.

Besonders vorteilhaft ist hierbei das gesamte Schirmgehäuse für eine Kommunikationsstrahlung mit einer Frequenz von größer als 1,8 GHz transparaente ausgebildet. Vorzugsweise ist das gesamte Schirmgehäuse für eine Kommunikationsstrahlung mit einer Frequenz von größer als 1,9 GHz transparent ausgebildet. Vorzugsweise ist das gesamte Schirmgehäuse für eine Kommunikationsstrahlung mit einer Frequenz von größer als 2,0 GHz transparent ausgebildet. Vorzugsweise ist das gesamte Schirmgehäuse für eine Kommunikationsstrahlung mit einer Frequenz von größer als 2,1 GHz transparent ausgebildet.

Alternativ oder zusätzlich kann es in einer vorteilhaften Weiterbildung der Erfindung kann es vorgesehen sein, dass das Schirmgehäuse eine Schnittstelle und eine Antenne aufweist, wobei die Antenne mittels der Schnittstelle an das Schirmgehäuse anschließbar ist. Hierbei kann vorteilhaft das gesamte Schirmgehäuse undurchlässig für eine elektromagnetische Strahlung ausgebildet sein. Eine Kommunikationsstrahlung und/oder Kommunikationssignale, die beispielsweise eine Frequenz von größer als 1,8 GHz und bevorzugt von größer als 2,0 GHz aufweisen, kann und/oder können dabei vorteilhaft mittels der Schnittstelle und der Antenne ausgetauscht werden. Beispielweise können über WLAN-Signale Daten zwischen einer Magnetresonanzvorrichtung und einem innerhalb des Schirmgehäuses der Magnetresonanzschutzvorrichtung angeordneten mobilen Endgeräts ausgetauscht werden. Die Schnittstelle kann beispielweise ein USB-Anschluss, einen Mini-USB-Anschluss, einen Steckverbinder, einen Klinkenstecker usw. umfassen, mittels der die Antenne an das Schirmgehäuse anschließbar ist.

In einer vorteilhaften Weiterbildung der Erfindung kann es vorgesehen sein, dass das Schirmgehäuse eine Befestigungseinheit umfasst. Die Befestigungseinheit umfasst bevorzugt zumindest ein Befestigungselement, mittels dessen die Magnetresonanzschutzvorrichtung an definierten Positionen befestigt und/oder angeordnet werden kann. Dies kann beispielsweise ein Befestigungselement zur Befestigung der Magnetresonanzschutzvorrichtung innerhalb eines Patientenaufnahmebereichs der Magnetresonanzvorrichtung sein, um die Magnetresonanzschutzvorrichtung an definierten und/oder vorbestimmten Positionen innerhalb des Patientenaufnahmebereichs während einer Magnetresonanzuntersuchung anzubringen. Das Befestigungselement kann beispielsweise ein Rastelement und/oder ein Klettverschlusselement und/oder weitere, dem Fachmann als sinnvoll erscheinende Befestigungselemente umfassen. Hierdurch kann eine komfortable Anordnung der Magnetresonanzschutzvorrichtung während der Magnetresonanzuntersuchung an der Magnetresonanzvorrichtung erreicht werden.

In einer vorteilhaften Weiterbildung der Erfindung kann es vorgesehen sein, dass das Schirmgehäuse zwei zueinander klappbare Gehäuseschalen eine Verschlusseinheit umfasst. Hierdurch kann eine einfache Aufnahme eines mobilen Endgeräts innerhalb des von dem Schirmgehäuse umschlossenen Bereichs erfolgen. Zudem kann mittels der Verschlusseinheit ein vorteilhaftes Abdichten des Schirmgehäuses erreicht werden. Insbesondere können mittels der Verschlusseinheit die zwei zueinander klappbaren Gehäuseschalen in einem verschlossenen Zustand der Verschlusseinheit aneinander gedrückt werden, so dass eine Dichtigkeit hinsichtlich einer Durchlässigkeit von elektromagnetischer Strahlung mit einer Frequenz kleiner als 1,5 GHz erreicht werden kann.

Darüber hinaus geht die Erfindung aus von einem Magnetresonanzsystem mit einer Magnetresonanzvorrichtung und einer Magnetresonanzschutzvorrichtung.

Es kann vorteilhaft der von dem Schirmgehäuse umschlossene Bereich und damit auch ein mobiles Endgerät, das innerhalb des von dem Schirmgehäuse umschlossenen Bereichs angeordnet ist, für magnetresonanzsensitive Frequenzen abgeschirmt werden. Insbesondere blockiert und/oder verhindert das Schirmgehäuse eine Transmission von magnetresonanzsensitiven Frequenzen durch das Schirmgehäuse. Magnetresonanzsensitive Frequenzen sind dabei im Bereich von kleiner als 1,5 GHz, wie insbesondere kleiner als 1,3 GHz und bevorzugt kleiner als 1,1 GHz angeordnet.

Des Weiteren kann eine einfache Integration eines mobilen Endgeräts eines Benutzers in ein Magnetresonanzsystem ermöglicht werden. Insbesondere kann beispielsweise während einer Magnetresonanzuntersuchung der Patient sein mobiles Endgerät für eine Patientenunterhaltung und/oder zu einer Übermittlung von Anweisungen während der Magnetresonanzuntersuchung mit in den Untersuchungsraum nehmen. Dies kann auch zur Beruhigung des Patienten während der Magnetresonanzuntersuchung beitragen, da dem Patienten seine gewohnte Darstellung eines Unterhaltungsmediums zur Verfügung steht.

Zudem kann auch ein medizinisches Bedienpersonal sein individualisiertes mobiles Endgerät zur Kontrolle der Magnetresonanzuntersuchung mit in den Untersuchungsraum nehmen. Dies erleichtert beispielsweise einen Zugriff auf bereits voreingestellte und/oder vorinstallierte Einstellungen für einzelne Untersuchungsschritte. Hierdurch kann auch ein Untersuchungsablauf beschleunigt werden, da ein Zugriff auf personalisierte Einstellungen erleichtert werden kann.

Die Vorteile des erfindungsgemäßen Magnetresonanzsystems entsprechen im Wesentlichen den Vorteilen der erfindungsgemäßen Magnetresonanzschutzvorrichtung, welche vorab im Detail ausgeführt sind. Hierbei erwähnte Merkmale, Vorteile oder alternative Ausführungsformen können ebenso auch auf die anderen beanspruchten Gegenstände übertragen werden und umgekehrt.

Weitere Vorteile, Merkmale und Einzelheiten der Erfindung ergeben sich aus dem im Folgenden beschriebenen Ausführungsbeispiel sowie anhand der Zeichnungen.

Es zeigen:
- Fig. 1: eine erfindungsgemäße Magnetresonanzschutzvorrichtung in einer schematischen Darstellung,
- Fig. 2: ein Abschirmverhalten der Magnetresonanzschutzvorrichtung,
- Fig. 3: eine zu Fig. 1 alternative Ausgestaltung der Magnetresonanzschutzvorrichtung und
- Fig. 4: ein Magnetresonanzsystem mit der Magnetresonanzschutzvorrichtung in einer schematischen Darstellung.

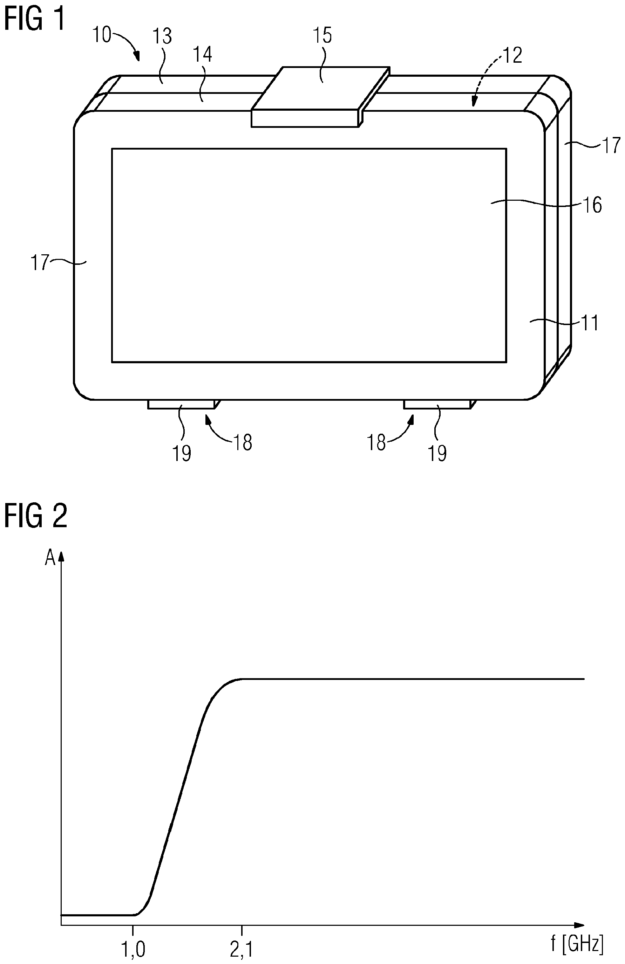

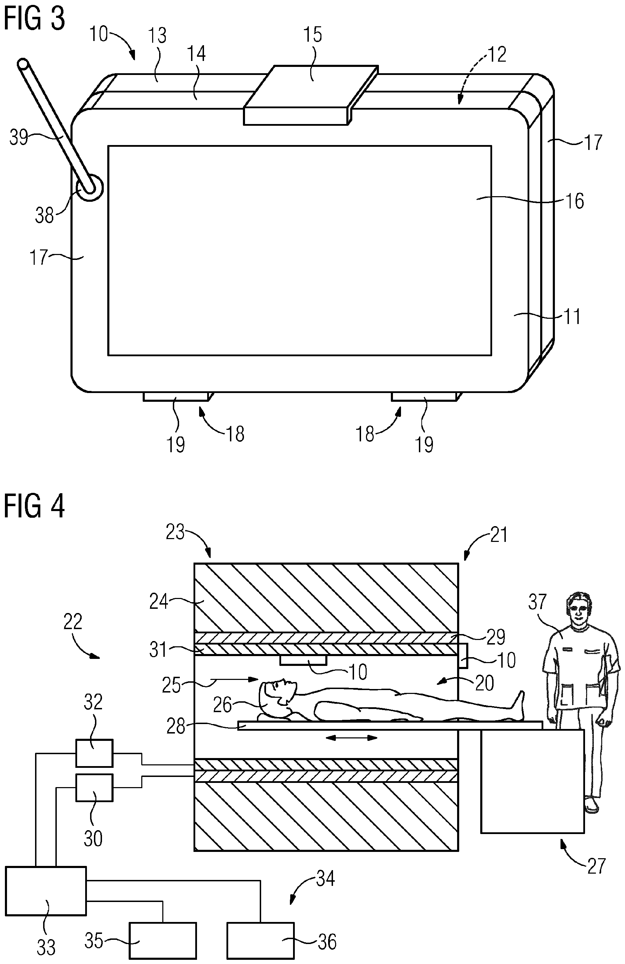

In den Fig. 1 und 3 ist jeweils eine erfindungsgemäße Magnetresonanzschutzvorrichtung 10 schematisch dargestellt. Die Magnetresonanzschutzvorrichtung 10 umfasst ein Schirmgehäuse 11 und einen von dem Schirmgehäuse 11 umschlossenen Bereich 12 und ist zur Aufnahme eines nicht näher dargestellten mobilen Endgeräts ausgelegt. Das Schirmgehäuse 11 ist hierbei für elektromagnetische Hochfrequenzstrahlung mit einer Frequenz f von kleiner als 1,5 GHz undurchlässig und/oder intransparent ausgebildet. Besonders vorteilhaft ist das Schirmgehäuse 11 für eine elektromagnetische Hochfrequenzstrahlung mit einer Frequenz f von kleiner als 1,4 GHz undurchlässig und/oder intransparent ausgebildet. Besonders vorteilhaft ist das Schirmgehäuse 11 für eine elektromagnetische Hochfrequenzstrahlung mit einer Frequenz f von kleiner als 1,3 GHz undurchlässig und/oder intransparent ausgebildet. Besonders vorteilhaft ist das Schirmgehäuse 11 für eine elektromagnetische Hochfrequenzstrahlung mit einer Frequenz f von kleiner als 1,2 GHz undurchlässig und/oder intransparent ausgebildet. Besonders vorteilhaft ist das Schirmgehäuse 11 für eine elektromagnetische Hochfrequenzstrahlung mit einer Frequenz f von kleiner als 1,1 GHz undurchlässig und/oder intransparent ausgebildet. Besonders vorteilhaft ist das Schirmgehäuse 11 für eine elektromagnetische Hochfrequenzstrahlung mit einer Frequenz f von kleiner als 1,0 GHz undurchlässig und/oder intransparent ausgebildet.

Das mobile Endgerät kann beispielsweise eine Smartphone und/oder eine Tablet-PC sein. Dabei kann das mobile Endgerät ein mobiles Endgerät eines Patienten 26 und/oder ein mobiles Endgerät eines medizinischen Bedienpersonals 37 umfassen.

Das Schirmgehäuse 11 weist zwei zueinander klappbare Gehäuseschalen 13, 14 und eine Verschlusseinheit 15 auf. Die Verschlusseinheit 15 kann dabei einen Klemmverschluss und/oder einen Rastverschluss und/oder weitere, dem Fachmann als sinnvoll erscheinenden Verschlusseinheiten 15 umfassen.

Die beiden zueinander klappbaren Gehäuseschalen 13, 14 können bei geöffneter Verschlusseinheit 15 geöffnet werden, insbesondere aufgeklappt werden. In diesem Zustand ist der von dem Schirmgehäuse 11 umschlossene Bereich 12 offen und es kann ein mobiles Endgerät in den von dem Schirmgehäuse 11 umschlossenen Bereich 12 eingebracht oder entfernt werden.

In einem geschlossenen Zustand der beiden Gehäuseschalen 13, 14 kann auch die Verschlusseinheit 15 geschlossen werden. In diesem Zustand umschließt dabei das Schirmgehäuse 11 den umschlossenen Bereich 12 komplett.

Das Schirmgehäuse 11 weist weiterhin einen optisch transparenten Bereich 16 und einen optisch nichttransparenten Bereich 17 auf. Der optisch transparente Bereich 16 ist im vorliegenden Ausführungsbeispiel auf eine einzige der beiden Gehäuseschalen 14 beschränkt, wobei diese Gehäuseschale 14 um den optisch transparenten Bereich 16 und einen optisch nichttransparenten Randbereich 17 aufweist.

Der optisch transparente Bereich 16 ist zur Abschirmung einer Bildschirmoberfläche des mobilen Endgeräts ausgelegt. Insbesondere wird das mobile Endgerät derart innerhalb des Schirmgehäuses 11, insbesondere des umschlossenen Bereichs 12, angeordnet, dass ein Bildschirm, insbesondere ein Display und/oder ein Touch-Display, direkt hinter dem optisch transparenten Bereich 16 angeordnet ist.

Der optisch transparente Bereich 16 ist bevorzugt für eine Strahlung, insbesondere eine elektromagnetische Strahlung, im für das menschliche Auge sichtbaren Bereich transparent und/oder durchlässig ausgebildet. Jedoch ist gleichzeitig der optisch transparente Bereich 16 für eine elektromagnetische Hochfrequenzstrahlung mit einer Frequenz f von kleiner als 1,5 GHz weiterhin undurchlässig und/oder intransparent ausgebildet.

Der optisch transparente Bereich 16 umfasst eine optisch transparente Folie. Zudem kann der optisch transparente Bereich auch eine metallische Gitterstruktur umfassen. Bevorzugt umfasst der optisch transparente Bereich eine optisch transparente Folie mit einer metallischen Gitterstruktur. Die metallische Gitterstruktur ist dabei derart ausgebildet, dass eine Durchlässigkeit und/oder eine Transmission für elektromagnetische Strahlung im Bereich des sichtbaren Lichts gegeben ist und für elektromagnetische Strahlung mit einer Frequenz f von kleiner als 1,5 GHz eine Transmission und/oder Durchlässigkeit blockiert ist.

Die Magnetresonanzschutzvorrichtung 10 in Fig. 1 ist dabei derart ausgebildet, dass der optisch transparente Bereich 16 ist zudem für eine Kommunikationsstrahlung mit einer Frequenz f größer als 1,8 GHz transparent und/oder durchlässig ausgebildet. Insbesondere ist hierbei der optisch transparente Bereich 16 für beispielsweise eine Bluetooth-Strahlung in einem Frequenzbereich von 2,4 GHz und/oder eine Wifi-Strahlung in einem Frequenzbereich von 2,4 GHz oder 5 GHz usw. transparent und/oder durchlässig ausgebildet.

Der optisch nichttransparente Bereich 17 der Magnetresonanzschutzvorrichtung 10 in Fig. 1 dagegen umfasst den Randbereich der Gehäuseschale 14 mit dem optisch transparenten Bereich 16. Zudem umfasst der optisch nichttransparente Bereich 17 auch die weitere Gehäuseschale 13 des Schirmgehäuses 11 komplett. Der optisch nicht transparente Bereich 17 ist bevorzugt für eine Strahlung, insbesondere eine elektromagnetische Strahlung, im für das menschliche Auge sichtbaren Bereich nichttransparent und/oder undurchlässig ausgebildet.

Im Ausführungsbeispiel der Fig. 1 umfasst auch der optisch nichttransparente und/oder optisch undurchlässige Bereich 17 des Schirmgehäuses 11 eine metallische Gitterstruktur. Die metallische Gitterstruktur des optisch nichttransparenten Bereichs 17 und/oder des optisch undurchlässigen Bereichs blockiert dabei sowohl eine Durchlässigkeit und/oder eine Transmission für elektromagnetische Strahlung im Bereich des sichtbaren Lichts als auch für elektromagnetische Strahlung mit einer Frequenz f von kleiner als 1,5 GHz.

Im Ausführungsbeispiel der Fig. 1 ist das gesamte Schirmgehäuse 11 derart ausgebildet, dass auch der optisch nichttransparente Bereich für eine Kommunikationsstrahlung mit einer Frequenz f größer als 1,8 GHz transparent und/oder durchlässig ausgebildet. Insbesondere ist hierbei der optisch nichttransparente Bereich 17 für beispielsweise eine Bluetooth-Strahlung in einem Frequenzbereich von 2,4 GHz und/oder eine Wifi-Strahlung in einem Frequenzbereich von 2,4 GHz oder 5 GHz usw. transparent und/oder durchlässig ausgebildet. Das Abschirmverhalten des Schirmgehäuses 11, insbesondere des optisch transparenten Bereichs 16 als auch des optisch nichttransparenten Bereichs 17, ist für Frequenzen f im GHz-Bereich in Fig. 2 dargestellt. Hierbei ist die Amplitude A eines Transmissionssignals über die Frequenz f dargestellt.

In Fig. 3 ist eine alternative Ausführung der Magnetresonanzschutzvorrichtung 10 dargestellt. Die Magnetresonanzschutzvorrichtung 10, insbesondere das gesamte Schirmgehäuse 11 der Magnetresonanzschutzvorrichtung 10, ist hierbei für elektromagnetische Strahlung undurchlässig und/oder nichttransparent ausgebildet, insbesondere für elektromagnetische Strahlung mit einer Frequenz f von kleiner 2 GHz. Hierbei kann das Schirmgehäuse 11 ebenfalls eine metallische Gitterstruktur umfassen.

Für eine Kommunikation eines innerhalb der Magnetresonanzschutzvorrichtung 10 angeordneten mobilen Endgeräts weist das Schirmgehäuse 11 in Fig. 3 eine Schnittstelle 38 und eine Antenne auf 39. Mittels der Schnittstelle 38 ist die Antenne 39 an das Schirmgehäuse 11 anschließbar für einen Austausch von Kommunikationssignalen zwischen beispielsweise dem mobilen Endgerät und der Magnetresonanzvorrichtung 21. Vorzugsweise können mittels der Schnittstelle und der Antenne Kommunikationssignale, die beispielsweise eine Frequenz von größer als 1,8 GHz und bevorzugt von größer als 2,0 GHz aufweisen, ausgetauscht werden. Beispielweise können über WLAN-Signale Daten zwischen der Magnetresonanzvorrichtung 21 und einem innerhalb des Schirmgehäuses 11 der Magnetresonanzschutzvorrichtung 10 angeordneten mobilen Endgeräts ausgetauscht werden. Die Schnittstelle 38 kann beispielweise ein USB-Anschluss, einen Mini-USB-Anschluss, einen Steckverbinder, einen Klinkenstecker usw. umfassen, mittels der die Antenne 39 an das Schirmgehäuse 11 anschließbar ist. Vorzugsweise weist die Antenne 39 einen zu der Schnittstelle 38 korrespondierenden Anschluss auf.

Das Schirmgehäuse 11 in den Ausführungsbeispielen der Fig. 1 und 3 weist weiterhin eine Befestigungseinheit 18 mit zumindest einem Befestigungselement 19 auf. Im vorliegenden Ausführungsbeispiel weist die Befestigungseinheit 18 zwei Befestigungselemente 19 auf, die gleichartig ausgebildet sind. Die Befestigungseinheit 18, insbesondere die Befestigungselemente 19, ist dabei zu einer Befestigung und/oder Anordnung der Magnetresonanzschutzvorrichtung 10 an weiteren Einheiten ausgelegt. Beispielsweise kann mittels der Befestigungseinheit 18 die Magnetresonanzschutzvorrichtung 10 für eine Patientenunterhaltung während einer Magnetresonanzuntersuchung innerhalb eines Patientenaufnahmebereichs 20 einer Magnetresonanzvorrichtung 21 angeordnet werden. Zudem kann auch mittels der Befestigungseinheit 18 die Magnetresonanzschutzvorrichtung 10 während einer Patientenvorbereitung an einem Gehäuse der Magnetresonanzvorrichtung 21 angeordnet werden. Die einzelnen Befestigungselemente 19 können dabei von Rastelementen und/oder von Klettverschlüssen usw. gebildet sein.

In Fig. 4 ist ein Magnetresonanzsystem 22 mit einer Magnetresonanzvorrichtung 21 und einer Magnetresonanzschutzvorrichtung 10 dargestellt. Die Magnetresonanzvorrichtung 21 umfasst eine Magneteinheit 23, die einen supraleitenden Hauptmagneten 24 zu einem Erzeugen eines starken und insbesondere konstanten Hauptmagnetfelds 25 umfasst. Zudem weist die Magnetresonanzvorrichtung 21 den Patientenaufnahmebereich 20 auf zu einer Aufnahme eines Patienten 26. Der Patientenaufnahmebereich 20 im vorliegenden Ausführungsbeispiel ist zylinderförmig ausgebildet und in einer Umfangsrichtung von der Magneteinheit 23 zylinderförmig umgeben. Grundsätzlich ist jedoch eine davon abweichende Ausbildung des Patientenaufnahmebereichs 20 jederzeit denkbar. Der Patient 26 kann mittels einer Patientenlagerungsvorrichtung 27 der Magnetresonanzvorrichtung 21 in den Patientenaufnahmebereich 20 geschoben und/oder gefahren werden. Die Patientenlagerungsvorrichtung 27 weist hierzu einen innerhalb des Patientenaufnahmebereichs 20 bewegbar ausgestalteten Patiententisch 28 auf.

Die Magneteinheit 23 weist weiterhin eine Gradientenspuleneinheit 29 zu einer Erzeugung von Magnetfeldgradienten auf, die für eine Ortskodierung während einer Bildgebung verwendet werden. Die Gradientenspuleneinheit 29 wird mittels einer Gradientensteuereinheit 30 der Magnetresonanzvorrichtung 21 gesteuert. Die Magneteinheit 23 umfasst weiterhin eine Hochfrequenzantenneneinheit 31 zu einer Anregung einer Polarisation, die sich in dem von dem Hauptmagneten 24 erzeugten Hauptmagnetfeld 25 einstellt. Die Hochfrequenzantenneneinheit 31 wird von einer Hochfrequenzantennensteuereinheit 32 der Magnetresonanzvorrichtung 21 gesteuert und strahlt hochfrequente Magnetresonanzsequenzen in einen Untersuchungsraum, der im Wesentlichen von einem Patientenaufnahmebereich 20 der Magnetresonanzvorrichtung 21 gebildet ist, ein.

Zu einer Steuerung des Hauptmagneten 23, der Gradientensteuereinheit 30 und zur Steuerung der Hochfrequenzantennensteuereinheit 32 weist die Magnetresonanzvorrichtung 21 eine Systemsteuereinheit 33 auf. Die Systemsteuereinheit 33 steuert zentral die Magnetresonanzvorrichtung 21, wie beispielsweise das Durchführen einer vorbestimmten bildgebenden Gradientenechosequenz. Zudem umfasst die Systemsteuereinheit 33 eine nicht näher dargestellte Auswerteeinheit zu einer Auswertung von medizinischen Bilddaten, die während der Magnetresonanzuntersuchung erfasst werden.

Des Weiteren umfasst die Magnetresonanzvorrichtung 21 eine Benutzerschnittstelle 34, die mit der Systemsteuereinheit 33 verbunden ist. Steuerinformationen wie beispielsweise Bildgebungsparameter, sowie rekonstruierte Magnetresonanzbilder können auf einer Anzeigeeinheit 35, beispielsweise auf zumindest einem Monitor, der Benutzerschnittstelle 34 für ein medizinisches Bedienpersonal angezeigt werden. Weiterhin weist die Benutzerschnittstelle 34 eine Eingabeeinheit 36 auf, mittels der Informationen und/oder Parameter während eines Messvorgangs von dem medizinischen Bedienpersonal eingegeben werden können.

Im vorliegende Ausführungsbeispiel umfasst das Magnetresonanzsystem 33 zwei Magnetresonanzschutzvorrichtungen 10, wobei die Magnetresonanzschutzvorrichtungen 10 nach dem Ausführungsbeispiel in Fig. 1 oder nach dem Ausführungsbeispiel in Fig. 3 ausgebildet sein können. Eine erste Magnetresonanzschutzvorrichtung 10 ist innerhalb des Patientenaufnahmebereichs 20 angeordnet für eine Unterhaltung des Patienten 26 während der Magnetresonanzuntersuchung. Das innerhalb der ersten Magnetresonanzschutzvorrichtung 10 angeordnete mobile Endgerät ist dabei von einem mobilen Endgerät des Patienten 26 gebildet.

Eine zweite Magnetresonanzschutzvorrichtung 10 ist an einer Frontseite der Magnetresonanzvorrichtung 21 angeordnet, um das medizinische Bedienpersonal 37 während der Magnetresonanzuntersuchung zu unterstützen. Das innerhalb der zweiten Magnetresonanzschutzvorrichtung 10 angeordnete mobile Endgerät ist dabei von einem mobilen Endgerät des medizinischen Bedienpersonals 37 gebildet.

Die erste Magnetresonanzschutzvorrichtung 10 und auch die zweite Magnetresonanzschutzvorrichtung 10 sind dabei jeweils mittels der Befestigungseinheit 18, insbesondere mittels der beiden Befestigungselemente 19, an der Magnetresonanzvorrichtung 21 angeordnet. Insbesondere sind die beiden Magnetresonanzschutzvorrichtung 10 mittels der Befestigungseinheit 18 lösbar an der Magnetresonanzvorrichtung 21 angeordnet.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

## Patentansprüche

1. Magnetresonanzschutzvorrichtung mit einem Schirmgehäuse und einen von dem Schirmgehäuse umschlossenen Bereich, der zu einer Aufnahme eines mobilen Endgeräts ausgelegt ist, **dadurch gekennzeichnet, dass** das Schirmgehäuse für elektromagnetischer Hochfrequenzstrahlung mit einer Frequenz von kleiner als 1,5 GHz undurchlässig und/oder nichttransparent ausgebildet ist.

2. Magnetresonanzschutzvorrichtung nach Anspruch 1, **dadurch gekennzeichnet, dass** das Schirmgehäuse den umschlossenen Bereich komplett umhüllt.

3. Magnetresonanzschutzvorrichtung nach einem der vorhergehenden Ansprüche,
**dadurch gekennzeichnet, dass** das Schirmgehäuse einen optisch transparenten Bereich und einen optisch nichttransparenten Bereich umfasst.

4. Magnetresonanzschutzvorrichtung nach Anspruch 3, **dadurch gekennzeichnet, dass** der optisch transparente Bereich zur Abschirmung einer Bildschirmoberfläche des mobilen Endgeräts ausgelegt ist.

5. Magnetresonanzschutzvorrichtung nach einem der Ansprüche 3 bis 4,
**dadurch gekennzeichnet, dass** der optisch transparente Bereich eine optisch transparente Folie umfasst.

6. Magnetresonanzschutzvorrichtung nach einem der Ansprüche 3 bis 5,
**dadurch gekennzeichnet, dass** der optisch transparente Bereich eine metallische Gitterstruktur umfasst.

7. Magnetresonanzschutzvorrichtung nach einem der vorhergehenden Ansprüche,
**dadurch gekennzeichnet, dass** das gesamte Schirmgehäuse eine metallische Gitterstruktur umfasst.

8. Magnetresonanzschutzvorrichtung nach einem der Ansprüche 3 bis 7,
**dadurch gekennzeichnet, dass** der optisch transparente Bereich für eine Kommunikationsstrahlung mit einer Frequenz größer als 1,8 GHz transparent ausgebildet ist.

9. Magnetresonanzschutzvorrichtung nach einem der vorhergehenden Ansprüche,
**dadurch gekennzeichnet, dass** das gesamte Schirmgehäuse für eine Kommunikationsstrahlung mit einer Frequenz größer als 1,8 GHz transparent ausgebildet ist.

10. Magnetresonanzschutzvorrichtung nach einem der vorhergehenden Ansprüche,
**dadurch gekennzeichnet, dass** das Schirmgehäuse eine Schnittstelle und eine Antenne aufweist, wobei die Antenne mittels der Schnittstelle an das Schirmgehäuse anschließbar ist.

11. Magnetresonanzschutzvorrichtung nach einem der vorhergehenden Ansprüche,
**dadurch gekennzeichnet, dass** das Schirmgehäuse eine Befestigungseinheit umfasst.

12. Magnetresonanzschutzvorrichtung nach einem der vorhergehenden Ansprüche,
**dadurch gekennzeichnet, dass** das Schirmgehäuse zwei zueinander klappbare Gehäuseschalen und eine Verschlusseinheit umfasst.

13. Magnetresonanzsystem mit einer Magnetresonanzvorrichtung und einer Magnetresonanzschutzvorrichtung nach einem der Ansprüche 1 bis 12.
